# FASCICULE DE BREVET EUROPEEN

(11) **EP 1 455 738 B1**
(45) Date de publication et mention de la délivrance du brevet: **28.07.2010**
(21) Numéro de dépôt: 02805371.8
(22) Date de dépôt: 12.12.2002
(51) Int. Cl.: A61K 8/44, A61Q 5/12

(54) **UTILISATION POUR LE TRAITEMENT DES CHEVEUX DE MONOMERES ELECTROPHILES**
VERWENDUNG VON ELEKTROPHILEN MONOMEREN FÜR HAARBEHANDLUNG
USE OF ELECTROPHILIC MONOMERS FOR HAIR TREATMENT

(30) Priorité: 18.12.2001 FR 0116402
(43) Date de publication de la demande: 15.09.2004
(73) Titulaire: L'Oréal, 75008 Paris (FR)
(72) Inventeur: GIROUD, Franck, F-92110 Clichy (FR)
(74) Mandataire: Casalonga, Axel
(86) Numéro de dépôt international: PCT/FR2002/004301
(87) Numéro de publication internationale: WO 2003/053380

(56) Documents cités:
- FR-A- 2 780 280
- US-A- 3 634 022
- US-A- 5 362 486
- US-A- 5 767 152
- US-A- 5 868 145
- V. BÖLLERT ET AL.: "Polymerisation von quaternisiertem 4-Vinylpyridin auf Humanhaar" PROCEEDINGS-INTERNATIONALE WOLLTEXTIL-FORSCHUNGSKONFERENZ, 5TH (1976), vol. 3, 1976, pages 403-415, XP001122897 Aachen, DE
- CIBA SPECIALTY CHEM. INC.: "Use of benzotriazole UV-absorbers for protection of hair" RESEARCH DISCLOSURE, septembre 2001 (2001-09), pages 1460-1464, XP001128228
- WIKIPEDIA: "Cyanoacrylate" [retrieved on 2007-09-07]

## Description

La présente invention concerne l'utilisation pour le traitement des cheveux, de compositions à base de monomères polymérisables in situ, ainsi que le procédé de traitement cosmétique correspondant.

On utilise généralement des compositions cosmétiques à base de silicones ou de polymères ayant une forte affinité pour les cheveux, en vue de modifier leurs propriétés superficielles, notamment pour les conditionner.

Il est généralement nécessaire de renouveler ces traitements dans la mesure où les agents de conditionnement ont tendance à s'éliminer, notamment aux shampoings.

Il est théoriquement possible d'accroître la rémanence du dépôt de polymères en effectuant directement une polymérisation radicalaire de certains monomères au niveau des cheveux.

Toutefois, les traitements ainsi obtenus sont inacceptables au point de vue cosmétique. On constate généralement une forte dégradation de la fibre liée probablement aux amorceurs de polymérisation et les cheveux traités sont difficilement démêlables.

Le document US 3,634,022 porte sur un procédé de mise en forme de substances kératiniques utilisant des composés insaturés oléfiniques dans le cadre d'une polymérisation radicalaire.

Le document FR 2 780 280 porte sur des compositions cosmétiques à usage topique comprenant au moins un dérivé de benzazole N-substitué particulier et au moins un polymère épaississant.

Le document US 5,767,152 décrit l'utilisation du dérivé d'acide cyanocarboxylique pour une application thérapeutique ou médicale de la repousse du poils ou du cheveu.

La demanderesse vient de découvrir qu'il était possible d'obtenir un conditionnement amélioré de la surface des cheveux et de façon durable, en mettant en oeuvre des monomères électrophiles pour lesquels un processus de polymérisation anionique s'amorce en présence d'un agent nucléophile tel que des ions hydroxyde (OH⁻) contenus dans l'eau à pH neutre.

La demanderesse a plus particulièrement constaté qu'en appliquant une composition à base de tels monomères sur la chevelure, il se forme in situ un polymère. Sans que cette explication soit limitative, il semble que ce soit les ions hydroxyde contenus dans l'eau absorbée par les cheveux qui enclenchent le processus de polymérisation anionique à l'interface composition de traitement-cheveux. Le polymère ainsi formé in situ par polymérisation anionique interfaciale se présente sous forme d'un dépôt homogène et possède une excellente adhésion aux cheveux.

Par ailleurs, on a constaté de façon surprenante que les cheveux restaient parfaitement individualisés et pouvaient être coiffés sans problème et que le conditionnement de la surface de la fibre est rémanente aux shampooings.

L'invention a donc pour premier objet l'utilisation de compositions comprenant au moins un monomère capable de polymériser par voie anionique en présence d'un agent nucléophile pour le traitement des cheveux, notamment pour le conditionnement de la surface des cheveux.

L'invention a également pour second objet un procédé mettant en oeuvre une composition utilisée dans le cadre de ce traitement.

D'autres objets de l'invention apparaîtront à la lecture de la description et des exemples qui suivent.

Par polymérisation anionique, on entend le mécanisme défini dans l'ouvrage "Advanced Organic Chemistry", Third Edition de Jerry March, pages 151 à 161.

Les monomères utilisés plus particulièrement conformément à l'invention sont les monomères de structure : 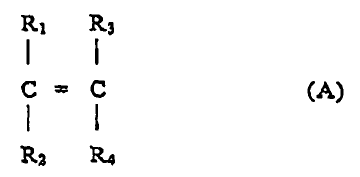 dans laquelle :
R₁ et R₂ désignent indépendamment l'un de l'autre des groupes peu ou non électro-attracteurs tels que :
   - un atome d'hydrogène,
   - un radical hydrocarboné saturé ou non, linéaire, ramifié ou cyclique, constitué de 4 à 20 atomes de carbone et pouvant contenir un ou plusieurs atomes d'azote, d'oxygéne, de soufre, et pouvant être éventuellement substitué par un ou plusieurs groupements choisi parmi OR, COOR, COR, SH, SR, OH
   - un résidu poly-siloxane organo modifié ou non,
   - un groupement polyoxyalkylène,
   - OR, COOR, COR, SH, SR, OH.
R₃ et R₄ désignent indépendamment l'un de l'autre des groupes électro-attracteurs choisis de préférence parmi les groupements N(R)₃+, S(R)₂+, SH₂+, NH₃+, NO₂, SO₂R, C≡N, COOH, F, CI, Br, I, OR, COOR, COR, SH, SR, OH, les radicaux alcényle linéaires ou ramifiés, les radicaux alcynyle linéaires ou ramifiés, les groupements mono- ou poly fluoroalkyle.
R désignant un radical alkyle en C₁-C₁₀ ou mono ou poly fluoroalkyle en C₁-C₁₀ et les groupes alkyle ont de 1 à 10 atomes de carbone. Les groupements alcényle ou alcynyle ont de préférence 2 à 10 atomes de carbone.

Parmi les polysiloxanes organo modifiés, on peut citer les polydiméthylsiloxanes à fonctions alkyle C₂-C₃₀ et/ou phényl et/ou siloxy et/ou silanol et/ou amine et/ou imine et/ou fluoroalkyl.

Parmi les groupements mono- ou poly fluoroalkyl, on peut citer des groupements tels que (CH₂)ₙ-(CF₂)ₘ-CF₃ ou (CH₂)ₙ-(CF₂)ₘ-CHF₂ avec n=1 à 20 et m= 1 à 20.

Parmi les groupements polyoxyalkylène, on peut citer les groupements polyoxyéthylène et les groupements polyoxypropylène ayant de préférence 1 à 200 unités oxyalkylénées.

Les substituants R₁ à R₄ peuvent éventuellement être substitués par un groupement ayant une activité cosmétique. Les activités cosmétiques particulièrement utilisées sont les fonctions colorantes, antioxydantes, filtres UV et conditionnantes.

Parmi les monomères précédemment cités, sont préférés les monomères de la famille des cyanoacrylates et leurs dérivés de formule (B) :

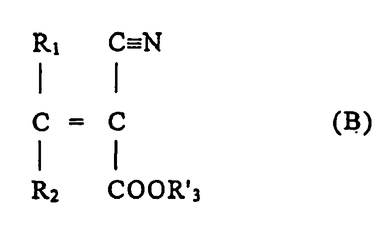

R₁ et R₂ ayant les mêmes significations que précédemment R'₃ pouvant désigner un groupe non électro-attracteur ou électro-attracteur tel que ceux définis pour la formule (A).

A titre de composés de formule (B), on peut citer les monomères constitués par la famille des fluoroalkyl-2-cyanoacrylates tels que :
a) 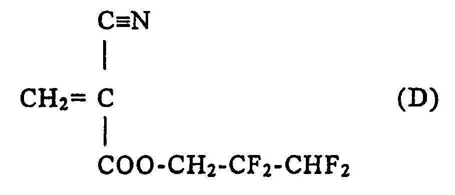 Le 2,2,3,3-tetrafluoropropyl ester de l'acide 2-cyano, 2-propenoïque, , ou encore : 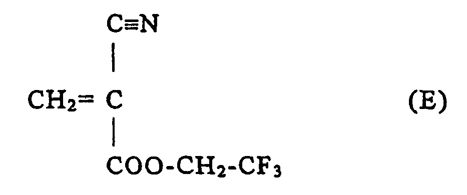 2,2,2 trifluoroéthyl ester de l'acide 2-cyano-, 2-propenoïque. ou
b)les alkyl ou alcoxy (C₁-C₁₀) cyanoacrylates et leurs dérivés.

On peut citer plus particulièrement : l'éthyl 2-cyanoacrylàte, le méthyl 2-cyanoacrylate, le n-propyl 2-cyanoacrylate, l'isopropyl 2-cyanoacrylate, le tert-butyl 2-cyanoacrylate, le n-butyl 2-cyanoacrylate, l'iso-butyl 2-cyanoacrylate, le 3-methoxybutyl cyanoacrylate, le n-decyl cyanoacrylate, l'hexyl 2-cyanoacrylate, le 2-éthoxyéthyl 2-cyanoacrylate, le 2-méthoxyéthyl 2-cyanoacrylate, le 2-octyl 2-cyanoacrylate, le 2-propoxyéthyl 2-cyanoacrylate, n-octyl 2-cyanoacrylate et l'iso amyl cyanoacrylate.

Dans le cadre de l'invention, on préfère utiliser les monomères b).

Le monomère le plus particulièrement préféré est le n-octyl 2-cyanoacrylate :

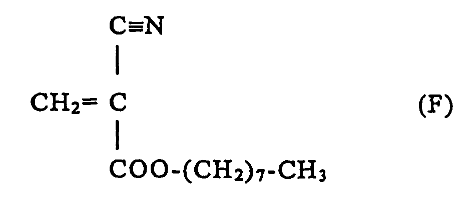

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Les agents nucléophiles susceptibles d'initier la polymérisation anionique sont des systèmes connus en eux-mêmes, capables de générer un carbanion au contact d'un agent nucléophile, tels que les ions hydroxydes contenus dans l'eau à pH neutre. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C⁼C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻, H₂O, etc.

On utilise de préférence un milieu anhydre et non hygroscopique pour véhiculer les monomères de l'invention, qui peut être utilisé pur ou sous forme d'une émulsion ou être encapsulé. On entend par « milieu anhydre », un milieu contenant moins de 1% d'eau,

Le milieu cosmétiquement acceptable contenant les monomères précités est de préférence choisi parmi les huiles organiques, les silicones, les huiles minérales, les cires, ou encore des solvants organiques tels que des alcanes en C₅-C₁₀, l'acétone, la méthyl éthyl cétone, l'acétate de méthyle, l'acétate de butyle, l'acétate d'éthyle, le diméthoxyéthane, le diéthoxyéthane, les alcools gras, les acides gras, les esters gras, les amides gras, les benzoates d'alcool gras et leurs mélanges.

Les compostions mises en oeuvre conformément à l'invention ont généralement une concentration en monomère selon l'invention comprise entre 0,001 et 50% en poids, et plus particulièrement entre 0,1 et 10% en poids.

On peut également introduire dans les compositions des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, les acides organiques tels que l'acide sulfonique ou l'acide phosphorique, le trifluorure de bore, l'hydroquinone et ses dérivés tels que l'hydroquinone monéthyléther, la TBHQ, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges. Les groupements alkyle désignent de préférence des groupement ayant 1 à 6 atomes de carbone.

La concentration en inhibiteur dans la composition de l'invention peut être comprise entre 10 ppm et 5%, et plus préférentiellement entre 10 ppm et 0,5% en poids.

Les compositions conformes à l'invention peuvent également contenir un propulseur qui peut être constitué par des gaz comprimés ou liquéfiés usuellement employés pour la préparation de compositions aérosols. On utilisera de préférence l'air, le gaz carbonique, l'azote comprimé ou encore un gaz soluble tel que le diméthyléther, les hydrocarbures halogénés tels que fluorés ou non, et leurs mélanges.

Les compositions conformes à l'invention peuvent également contenir des agents utilisés habituellement en cosmétique, tels que des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, des agents nacrants, etc.

Le procédé de traitement des cheveux conforme à l'invention consiste à appliquer la composition décrite ci-dessus sur les cheveux et en particulier en présence d'un agent nucléophile.

De préférence, cet agent nucléophile est l'eau. Cette eau peut être apportée par une humidification préalable.

Il est également possible, afin de moduler la cinétique de réaction, de préalablement humidifier la fibre à l'aide d'une solution aqueuse dont le pH a été ajusté à l'aide d'une base, d'un acide ou d'un mélange acide/base. L'acide et/ou la base peuvent être inorganique ou organique.

Il est également possible de moduler la cinétique de polymérisation par voie anionique en pré-imprégnant la fibre à l'aide d'un agent nucléophile. L'agent nucléophile peut être utilisé pur, en solution, sous forme d'une émulsion ou être encapsulé.

Pour moduler la cinétique de polymérisation anionique, on peut également augmenter la nucléophilie de la fibre par transformation chimique de la matière kératinique. A titre d'exemple, on peut citer la réduction des ponts di-sulfure composant en partie la kératine en thiols avant application de la composition de l'invention, de façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le polyméthylméthacrylate (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter la fibre avec tous types de polymères, ou réaliser un traitement capillaire avant application de la composition de l'invention, comme une coloration directe ou d'oxydation, une permanente ou encore un défrisage.

L'application des compositions conformes à l'invention peut être suivie ou non d'un rinçage. Ces compositions peuvent se présenter sous des formes diverses, telles que de lotion, de spray, de mousse et être appliquées sous forme de shampooing ou d'après-shampooing.

Les exemples qui suivent sont destinés à illustrer l'invention, sans présenter un caractère limitatif.

### EXEMPLE 1

On a préparé le composition suivante :

| | |
|---|---|
| n-Octyl 2-cyanoacrylate (1) | 10 g |
| Huile silicone (2) | 90 g |

- RITE LOK CON895, commercialisé par la Société CHEMENCE
- Dow Corning 556 Fluid Cosmetic

### Mode d'application 1

2 g de composition décrite ci-dessus sont appliqués sur une mèche constituée de 2,7 g de cheveux sensibilisés de solubilité alcaline égale à 20%. Après une minute de contact de la composition avec la mèche de cheveux, celle-ci est lavée à l'aide d'un shampooing DOP camomille, puis séchée 1 heure à l'ambiante.

La même opération est également réalisée sur une autre mèche avec l'huile silicone seule.

### Mode d'application 2

Une mèche constituée de 2,7 g de cheveux sensibilisés de solubilité alcaline égale à 20% est humidifiée à l'aide d' 1 ml d'eau. 2 g de composition décrite ci-dessus sont appliqués sur cette mèche humidifiée. Après une minute de contact de la composition avec la mèche de cheveux, celle-ci est lavée à l'aide d'un shampooing DOP camomille, puis séchée 1 heure à l'ambiante.

La même opération est également réalisée sur une autre mèche humidifiée avec l'huile silicone seule.

Pour chaque mèche, le toucher des cheveux est évalué par un panel de 10 personnes. Une mèche vierge de même solubilité alcaline est utilisée comme référence.

L'évaluation tactile des diverses mèches de cheveux est répétée avec la même procédure après avoir réalisé successivement 5 shampooings commercialisés sous la dénomination DOP camomille.

### Résultats

| | | Mode d'application n° 1 | | Mode d'application n° 2 | |
|---|---|---|---|---|---|
| | Nature du traitement | Composition | Silicone | Composition | Silicone |
| Toucher de la fibre | Après Application + shampooing | Gainé 3 Gras 4 | Gainé 0 Gras 5 | Gainé 4 Gras 3 | Gainé 0 Gras 4 |
| | Après 5 shampooings | Gainé 3 Gras 0 | Gainé 0 Gras 0 | Gainé 3 Gras 0 | Gainé 0 Gras 0 |

| | | | | | |
|---|---|---|---|---|---|
| Notation : 0 non détectable →5 très marqué | | | | | |

L'expérience montre que la modification tactile de la fibre apportée par le monomère selon l'invention (touché gainé) est rémanente aux shampooings alors que la modification du toucher apportée par la silicone (toucher gras) n'est pas rémanente aux shampooings. La composition permet donc de conserver, après plusieurs shampooings, le toucher modifié et ceci sans ré-application de la composition.

## Revendications

1. Utilisation pour le conditionnement de la surface des cheveux d'une composition contenant dans un milieu cosmétiquement acceptable au moins un monomère
choisi parmi les monomères de formule : 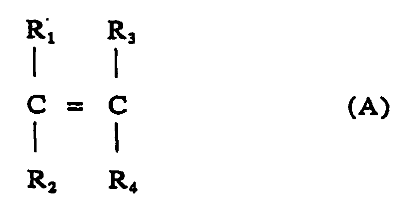 dans laquelle :
R₁ et R₂ désignent indépendamment l'un de l'autre des groupes peu ou non électro-attracteurs tels que :
- un atome d'hydrogène,
- un radical hydrocarboné saturé ou non, linéaire, ramifié ou cyclique, constitué de préférence de 4 à 20 atomes de carbone et pouvant contenir un ou plusieurs atomes d'azote, d'oxygène, de soufre, et pouvant être éventuellement substitués par un ou plusieurs groupements choisi parmi OR, COOR, COR, SH, SR, OH,
- un résidu poly-siloxane modifié organo ou non,
- un groupement polyoxyalkylène,
- OR, COOR, COR, SH, SR, OH.
R₃ et R₄ désignent indépendamment l'un de l'autre des groupes électro-attracteurs choisis parmi les groupements N(R)₃+, S(R)₂+, SH₂+, NH₃+, NO₂, SO₂R, C≡N, COOH, F, CI, Br, I, OR, COOR, COR, SH, SR, OH; les radicaux alcényle linéaires ou ramifiés, les radicaux alcynyle linéaires ou ramifiés, les groupements mono ou poly fluoroalkyle, R désignant un radical alkyle en C1-C10 ou mono ou polyfluoroalkyle en C1-C10 capable de polymériser par polymérisation anionique en présence d'un agent nucléophile, la concentration en monomère étant comprise entre 0,1 et 10% en poids.

2. Utilisation selon le revendication 1 **caractérisée par le fait que** le ou les monomères sont choisis parmi les composés de formule : 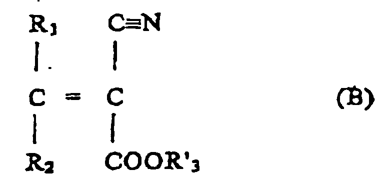 R₁ et R₂ ayant les mêmes significations que précédemment R'₃ pouvant désigner un groupe non électro-attracteur ou électro-attracteur tel que ceux définis pour la formule (A).

3. Utilisation selon l'une quelconque dcs revendications 1 à 2 **caractérisée par le fait que** les monomères sont choisis parmi les alkyl ou alcoxy(Cl- C10)cyanoacrylates.

4. Utilisation selon l'une quelconque des revendications 1 à 3 **caractérisée par le fait que** les monomères sont choisis parmi l'éthyl 2-cyanoacrylate, le methyl 2-cyanoacrylate, le n-propyl 2-cyanoacrylate, l'isopropyl 2-cyanoacrylate, le tert-butyl 2-cyanoacrylate, le n-butyl 2-cyanoacrylate, l'iso-butyl 2-cyanoacrylate, le 3-methoxybutyl cyanoacrylate, le n-deeyl cyanoacrylate, l'hexyl 2-cyanoacrylate, le 2-éthoxyéthyl 2-cyanoacrylate, le 2-méthoxyéthyl 2-cyanoacrylate, le 2-octyl 2-cyanoacrylate, le 2-propoxyéthyl 2-cyanoacrylate, n-octyl 2-cyanoacrylate et l'iso amyl cyanoacrylate.

5. Utilisation selon l'une des revendications 1 a 4 **caractérisée par le fait que** le monomère est le n-octyl 2-cyanoacrylate.

6. Utilisation selon l'une quelconque des revendications 1 à 5 **caractérisée par le fait que** les monomères sont fixés de façon covalente sur des supports tels que des polymères, des oligomères ou des dendrimères.

7. Utilisation pour la traitement cheveux d'une composition **caractérisée par le fait qu'**elle contient dans un milieu cosmétiquement acceptable anhydre et non hygroscopique au moins un monomère capable de polymériser par polymérisation anionique tel que définit à la revendication 1 en présence d'un agent nucléophile.

8. Utilisation selon la revendication 7 **caractérisé par le fait que** le milieu cosmétiquement acceptable est choisi parmi les huiles organiques, les silicones, les huiles minérales, les cires, ou encore des solvants organiques tels que des alcanes en C₅-C₁₀, l'acétone, la méthyl éthyl cétone, l'acétate de méthyle, l'acétate de butyle, l'acétate d'éthyle, le diméthoxyéthane, le diéthoxyéthane, les alcools gras, les acides gras, les esters gras, les amides gras, les benzoates d'alcool gras et leurs mélanges.

9. Utilisation d'une composition selon la revendication 7 ou 8 **caractérisée par le fait qu'**elle contient des inhibiteurs de polymérisation.

10. Utilisation selon la revendication 9 **caractérisée par le fait que** les inhibiteurs sont des inhibiteurs de polymérisation anioniques et/ou radicalaires.

11. Utilisation selon la revendication 9 ou 10 **caractérisée par le fait que** les inhibiteurs de polymérisation sont choisis parmi le dioxyde de soufre, l'oxyde nitrique, les acides organiques tels que l'acide sulfonique ou l'acide phosphorique, le trifluorure de bore, l'hydroquinone et ses dérivés tels que l'hydroquinone monéthyléther, la TBHQ, la benzoquinone et ses dérivés tels que la duroquinone, le catéchol et ses dérivés tels que le t-butyl catéchol et le methoxycatéchol, l'anisole et ses dérivés tels que le méthoxyanisole on l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges.

12. Utilisation selon L'une quelconque des revendications 9 à 11 **caractérisée par le fait que** les inhibiteurs de polymérisation sont présents dans des concentrations comprises entre 10 ppm et 5%, et plus préférentiellement entre 10 ppm et 0,5% en poids.

13. Utilisation selon l'une quelconque des revendications 7 à 12. **caractérisée par le fait que** la composition continent des agents utilisés habituellement en cosmétique, tels que des agents réducteurs, des corps gras, des plastifiants, des adoucissants, des agents anti-mousse, des agents hydratants, des pigments, des argiles, des charges minérales, des filtres UV, des colloïdes, minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, des agents nacrant.

14. Utilisation selon l'une quelconque des revendications 7 à 13 **caractérisée par le fait que** la composition est sous forme de lotion, de spray ou de mousse.

15. Utilisation selon l'une quelconque des revendications 7 à 14 **caractérisée par le fait que** la composition contient un propulseur qui peut être constitué par des gaz comprimés ou liquéfiés usuellement employés pour le préparation de compositions aérosols.

16. Procédé pour le conditionnement de la surface des cheveux **caractérisé par le fait que** l'on applique une composition selon l'une quelconque des revendication 7 à 15 en présence d'un agent ancléophile.

17. Procédé selon la revendication 16 **caractérisé par le fait que** les agents nucléophiles sont un composé moléculaire, un oligomère, un dendrimère ou un polymère possédant des fonctions nucléophiles choisies parmi: R₂N⁻, NH₂⁻, Ph₃C⁻, R₃C⁻, PhNH⁻, pyridine, ArS⁻, R-C⁼C⁻, RS⁻, SH⁻, RO⁻, R₂NH, ArO⁻, N₂⁻, OH⁻, ArNH₂, NH_{3,} I⁻, Br⁻, Cl⁻, RCOO⁻, SCN⁻, ROH, RSH, NCO⁻, CN⁻, NO₃⁻ ClO₄⁻, H₂O.

18. Procédé selon la revendication 16 ou 17 **caracterisé par le fait que** l'on applique la composition contenant le ou les monomères définis selon l'une quelconque des revendication 1 à 6 sur les cheveux préalablement humidifiés.

19. Procédé salon l'une quelconque des revendications 16 à 18 **caractérisé par le fait que** l'on applique la composition contenant le monomère sur des cheveux préalablement humidifïées à l'aide d'une solution aqueuse dont le pH a été ajusté à l'aide d'une base, d'un acide ou d'un mélange acide/base.

20. Procédé selon l'une quelconque des revendications 16 et 17 **caractérisé par le fait que** l'on rapplique la composition contenant le monomère sur des fibres on pré-imprégnant la fibre à l'aide d'un agent nucléophile autre que l'eau.

21. Procédé selon l'une quelconque des revendications 16 à 20 **caractérisé par le fait que** l'agent nucléophile est utilisé sous forme pure, en solution, sous forme d'une émulsion ou être encapsulé.

22. Procédé Selon l'une quelconque des revendications 16 à 21 **caractérisé par le fait que** l'application de la composition contenant le ou les monomères définis selon l'une quelconque des revendications 1 à 5 est suivie d'un pinçage.

## Claims

1. Use, for conditioning the hair surface, of a composition containing, in a cosmetically acceptable medium, at least one monomer chosen from the monomers of formula: 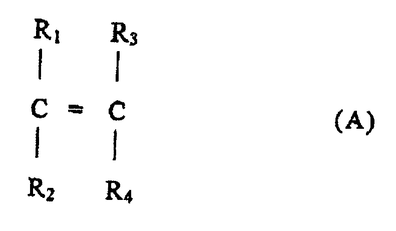 in which:
R₁ and R₂ denote, independently of each other, groups with little or no electron-withdrawing nature, such as:
- a hydrogen atom,
- a saturated or unsaturated, linear, branched or cyclic hydrocarbon-based radical, preferably containing from 4 to 20 carbon atoms and possibly containing one or more nitrogen, oxygen or sulphur atoms, and possibly being optionally substituted with one or more groups chosen from OR, COOR, COR, SH, SR and OH,
- an organomodified or non-organomodified poly-siloxane residue,
- a polyoxyalkylene group,
- OR, COOR, COR, SH, SR and OH;
R₃ and R₄ denote, independently of each other, electron-withdrawing groups chosen from N(R)₃+, S(R)₂+, SH₂+, NH₃+, NO₂, SO₂R, C≡N, COOH, F, Cl, Br, I, OR, COOR, COR, SH, SR and OH, linear or branched alkenyl radicals, linear or branched alkynyl radicals and mono- or polyfluoroalkyl groups, R denoting a C₁-C₁₀ alkyl or C₁-C₁₀ mono- or polyfluoroalkyl radical;
the said monomer being capable of polymerizing by anionic polymerization in the presence of a nucleophilic agent, the monomer concentration being between 0.1% and 10% by weight.

2. Use according to Claim 1, **characterized in that** the monomer(s) is(are) chosen from the compounds of formula: 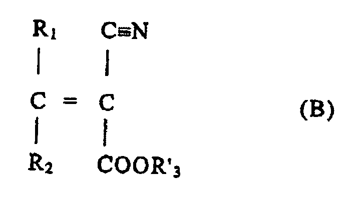 R₁ and R₂ having the same meanings as above, R'₃ possibly denoting a non-electron-withdrawing or electron-withdrawing group such as those defined for formula (A).

3. Use according to either of Claims 1 and 2, **characterized in that** the monomers are chosen from (C₁-C₁₀) alkyl or alkoxy cyanoacrylates.

4. Use according to any one of Claims 1 to 3, **characterized in that** the monomers are chosen from ethyl 2-cyanoacrylate, methyl 2-cyanoacrylate, n-propyl 2-cyanoacrylate, isopropyl 2-cyanoacrylate, tert-butyl 2-cyanoacrylate, n-butyl 2-cyanoacrylate, isobutyl 2-cyanoacrylate, 3-methoxybutyl cyanoacrylate, n-decyl cyanoacrylate, hexyl 2-cyanoacrylate, 2-ethoxyethyl 2-cyanoacrylate, 2-methoxyethyl 2-cyanoacrylate, 2-octyl 2-cyanoacrylate, 2-propoxyethyl 2-cyanoacrylate, n-octyl 2-cyanoacrylate and isoamyl cyanoacrylate.

5. Use according to one of Claims 1 to 4, **characterized in that** the monomer is n-octyl 2-cyanoacrylate.

6. Use according to any one of Claims 1 to 5, **characterized in that** the monomers are covalently attached to supports such as polymers, oligomers or dendrimers.

7. Use, for conditioning the hair surface, of a composition, **characterized in that** it contains, in a cosmetically acceptable anhydrous and non-hygroscopic medium, at least one monomer capable of polymerizing by anionic polymerization as defined in Claim 1 in the presence of a nucleophilic agent.

8. Use according to Claim 7, **characterized in that** the cosmetically acceptable medium is chosen from organic oils, silicones, mineral oils, waxes or organic solvents such as C₅-C₁₀ alkanes, acetone, methyl ethyl ketone, methyl acetate, butyl acetate, ethyl acetate, dimethoxyethane, diethoxyethane, fatty alcohols, fatty acids, fatty esters, fatty amides and fatty alkyl-benzoates, and mixtures thereof.

9. Use of a composition according to either of Claims 7 and 8, **characterized in that** it contains polymerization inhibitors.

10. Use according to Claim 9, **characterized in that** the inhibitors are free-radical and/or anionic polymerization inhibitors.

11. Use according to either of Claims 9 and 10, **characterized in that** the polymerization inhibitors are chosen from sulphur dioxide, nitric oxide, organic acids such as sulphonic acid or phosphoric acid, boron trifluoride, hydroquinone and its derivatives such as hydroquinone monoethyl ether, TBHQ, benzoquinone and its derivatives such as duroquinone, catechol and its derivatives such as t-butylcatechol and methoxycatechol, anisole and its derivatives such as methoxyanisole or hydroxyanisole, pyrogallol and its derivatives, p-methoxyphenol, hydroxybutyltoluene, alkyl sulphates, alkyl sulphites, alkyl sulphones, alkyl sulphoxides, alkyl sulphides, mercaptans and 3-sulphonene and mixtures thereof.

12. Use according to any one of Claims 9 to 11, **characterized in that** the polymerization inhibitors are present in concentrations of between 10 ppm and 5% and more preferably between 10 ppm and 0.5% by weight.

13. Use according to any one of Claims 7 to 12, **characterized in that** the composition contains agents usually used in cosmetics, such as reducing agents, fatty substances, plasticizers, softeners, antifoams, moisturizers, pigments, clays, mineral fillers, UV screening agents, mineral colloids, peptizers, solubilizing agents, fragrances, preserving agents, anionic, cationic, nonionic or amphoteric surfactants, fixing or non-fixing polymers, polyols, proteins, vitamins, direct dyes or oxidation dyes and nacreous agents.

14. Use according to any one of Claims 7 to 13, **characterized in that** the composition is in the form of a lotion, a spray or a mousse.

15. Use according to any one of Claims 7 to 14, **characterized in that** the composition contains a propellant which may consist of compressed or liquefied gases usually used for the preparation of aerosol compositions.

16. Process for conditioning the hair surface, **characterized in that** a composition according to any one of Claims 7 to 15 is applied in the presence of a nucleophilic agent.

17. Process according to Claim 16, **characterized in that** the nucleophilic agents are a molecular compound, an oligomer, a dendrimer or a polymer containing nucleophilic functions chosen from: R₂N⁻, NH₂⁻, Ph₃C⁻, R₃C⁻, PHNH⁻, pyridine, ArS⁻, R-C⁼C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻, H₂O.

18. Process according to either of Claims 16 and 17, **characterized in that** the composition containing the monomer(s) defined according to any one of Claims 1 to 6 is applied to hair that has been moistened beforehand.

19. Process according to any one of Claims 16 to 18, **characterized in that** the composition containing the monomer is applied to hair that has been moistened beforehand using an aqueous solution whose pH has been adjusted with the aid of a base, an acid or an acid/base mixture.

20. Process according to either of Claims 16 and 17, **characterized in that** the composition containing the monomer is applied to fibres by pre-impregnating the fibre using a nucleophilic agent other than water.

21. Process according to any one of Claims 16 to 20, **characterized in that** the nucleophilic agent is used in pure form, as a solution or in the form of an emulsion, or may be encapsulated.

22. Process according to any one of Claims 16 to 21, **characterized in that** the application of the composition containing the monomer(s) defined according to any one of Claims 1 to 5 is followed by rinsing.

## Patentansprüche

1. Verwendung einer Zusammensetzung, die in einem kosmetisch akzeptablen Medium mindestens ein Monomer enthält, das unter den Monomeren der folgenden Formel ausgewählt ist, zum Konditionieren der Oberfläche von Haaren: 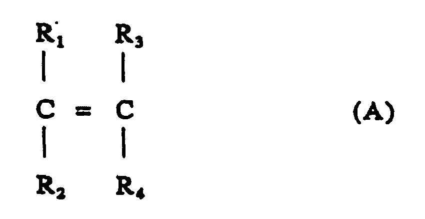 in der Formel:
R₁ und R₂ bedeuten unabhängig voneinander eine wenig oder nicht elektronenziehende Gruppe, wie:
- ein Wasserstoffatom,
- eine gesättigte oder ungesättigte, lineare, verzweigte oder cyclische Kohlenwasserstoffgruppe, die vorzugsweise aus 4 bis 20 Kohlenstoffatomen aufgebaut ist und ein oder mehrere Stickstoffatome, Sauerstoffatome und Schwefelatome enthalten kann und gegebenenfalls mit einer oder mehreren Gruppen substituiert sein kann, die unter OR, COOR, COR, SH, SR und OH ausgewählt sind,
- einen organomodifizierten oder nicht organomodifizierten Polysiloxanrest,
- eine Polyoxyalkylengruppe,
- OR, COOR, COR, SH, SR, OH,
R₃ und R₄ bedeuten unabhängig voneinander elektronenziehende Gruppen, die unter den Gruppen N(R)₃+, S(R)₂+, SH+, NH₃+, NO₂, SO₂R, C≡N, COOH, F, CI, Br, I, OR, COOR, COR, SH, SR, OH, geradkettigen oder verzweigten Alkenylgruppen, geradkettigen oder verzweigten Alkinylgruppen, Mono- oder Polyfluoralkylgruppen ausgewählt sind,
wobei R eine Alkyl(C₁₋₁₀)gruppe oder eine Mono- oder Polyfluoralkyl(C₁₋₁₀)gruppe bedeutet, die befähigt ist, über eine anionische Polymerisation in Gegenwart eines nucleophilen Stoffes zu polymerisieren, wobei die Konzentration des Monomers im Bereich von 0,1 bis 10 Gew.-% liegt.

2. Verwendung nach Anspruch 1, **dadurch gekennzeichnet, dass** das oder die Monomere unter den Verbindungen der folgenden Formel ausgewählt sind: 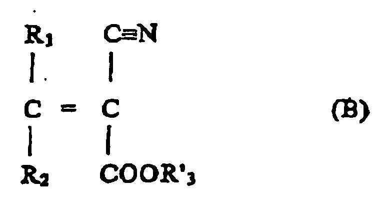 worin R₁ und R₂ die oben angegebenen Bedeutungen aufweisen und R'₃ eine nicht elektronenziehende Gruppe oder eine elektronenziehende Gruppe bedeuten kann, wie sie für die Formel (A) definiert wurden.

3. Verwendung nach einem der Ansprüche 1 bis 2, **dadurch gekennzeichnet, dass** die Monomere unter den Alkyl- oder Alkoxy(C₁₋₁₀)cyanoacrylaten ausgewählt sind.

4. Verwendung nach einem der Ansprüche 1 bis 3, **dadurch gekennzeichnet, dass** die Monomere unter Ethyl-2-cyanoacrylat, Methyl-2-cyanoacrylat, n-Propyl-2-cyanoacrylat, Isopropyl-2-cyanoacrylat, tert-Butyl-2-cyanoacrylat, n-Butyl-2-cyanoacrylat, Isobutyl-2-cyanoacrylat, 3-Methoxybutyl-cyanoacrylat, n-Decylcyanoacrylat, Hexyl-2-cyanoacrylat, 2-Ethoxyethyl-2-cyanoacrylat, 2-Methoxyethyl-2-cyanoacrylat, 2-Octyl-2-cyanoacrylat, 2-Propoxyethyl-2-cyanoacrylat, n-Octyl-2-cyanoacrylat und Isoamyl-2-cyanoacrylat ausgewählt sind.

5. Verwendung nach einem der Ansprüche 1 bis 4, **dadurch gekennzeichnet, dass** es sich bei dem Monomer um das n-Octyl-2-cyanoacrylat handelt.

6. Verwendung nach einem der Ansprüche 1 bis 5, **dadurch gekennzeichnet, dass** die Monomere kovalent an Träger gebunden sind, wie Polymere, Oligomere oder Dendrimere.

7. Verwendung einer Zusammensetzung für die Haarbehandlung, **dadurch gekennzeichnet, dass** sie in einem kosmetisch akzeptablen, wasserfreien und nicht hygroskopischen Medium mindestens ein Monomer enthält, das befähigt ist, wie in Anspruch 1 definiert, in Gegenwart eines nucleophilen Stoffes über eine anionische Polymerisation zu polymerisieren.

8. Verwendung nach Anspruch 7, **dadurch gekennzeichnet, dass** das kosmetisch akzeptable Medium unter den organischen Ölen, Siliconen, Mineralölen, Wachsen oder auch organischen Lösemitteln ausgewählt ist, wie C₅₋₁₀-Alkanen, Aceton, Methylethylketon, Methylacetat, Butylacetat, Ethylacetat, Dimethoxyethan, Diethoxyethan, Fettalkoholen, Fettsäuren, Fettsäureestern, Fettamiden, Fettalkoholbenzoaten und deren Gemischen.

9. Verwendung einer Zusammensetzung nach einem der Ansprüche 7 bis 8, **dadurch gekennzeichnet, dass** sie Polymerisationsinhibitoren enthält.

10. Verwendung nach Anspruch 9, **dadurch gekennzeichnet, dass** die Inhibitoren anionische und/oder radikalische Polymerisationsinhibitoren sind.

11. Verwendung nach einem der Ansprüche 9 bis 10, **dadurch gekennzeichnet, dass** die Polymerisationsinhibitoren unter Schwefeldioxid, Stickstoffmonoxid, organischen Säuren, wie Sulfonsäure oder Phosphorsäure, Bortrifluorid, Hydrochinon und seinen Derivaten, wie Hydrochinonmethylether, TBQH, Benzochinon und seinen Derivaten, wie Durochinon, Catechol und seinen Derivaten, wie tert-Butylcatechol und Methoxycatechol, Anisol und seinen Derivaten, wie Methoxyanisol oder Hydroxyanisol, Pyrogallol und seinen Derivaten, p-Methoxyphenol, Hydroxybutyltoluol, Alkylsulfaten, Alkylsulfiten, Alkylsulfonen, Alkylsulfoxiden, Alkylsulfiden, Mercaptanen, 3-Sulfonen und deren Gemischen ausgewählt sind.

12. Verwendung nach einem der Ansprüche 9 bis 11, **dadurch gekennzeichnet, dass** die Polymerisationsinhibitoren in Konzentrationen im Bereich von 10 ppm bis 5 % und vorzugsweise 10 ppm bis 0,5 Gew.-% vorliegen.

13. Verwendung nach einem der Ansprüche 7 bis 12, **dadurch gekennzeichnet, dass** die Zusammensetzung Stoffe enthält, die gewöhnlich in der Kosmetik eingesetzt werden, wie Reduktionsmittel, Fettsubstanzen, Weichmacher, reizlindernde Stoffe, Schaumverhütungsmittel, Hydratisierungsmittel, Pigmente, Tone, anorganische Füllstoffe, UV-Filter, anorganische Kolloide, peptisierende Stoffe, Lösemittel, Parfums, Konservierungsmittel, anionische, kationische, nichtionische oder amphotere grenzflächenaktive Stoffe, fixierende oder nicht fixierende Polymere, Polyole, Proteine, Vitamine, Direktfarbstoffe oder Oxidationsfarbstoffe und Perlglanzstoffe.

14. Verwendung nach einem der Ansprüche 7 bis 13, **dadurch gekennzeichnet, dass** die Zusammensetzung als Lotion, Spray oder Schaum vorliegt.

15. Verwendung nach einem der Ansprüche 7 bis 14, **dadurch gekennzeichnet, dass** die Zusammensetzung ein Treibmittel enthält, das aus Druckgasen oder Flüssiggasen gebildet ist, die gewöhnlich für die Herstellung von Aerosolen verwendet werden.

16. Verfahren zur Konditionierung der Haaroberfläche, **dadurch gekennzeichnet, dass** eine Zusammensetzung nach einem der Ansprüche 7 bis 15 in Gegenwart eines nucleophilen Stoffes verwendet wird.

17. Verfahren nach Anspruch 16, **dadurch gekennzeichnet, dass** es sich bei den nucleophilen Stoffen um eine molekulare Verbindung, ein Oligomer, ein Dendrimer oder ein Polymer handelt, die nucleophile Funktionen aufweisen, die ausgewählt sind unter: R₂N-, NH₂-, Ph₃C-, R₃C-, PhNH-, Pyridin, ArS-, R-C=C-, RS-, SH-, RO-, R₂NH, ArO-, N₃-, OH-, ArNH₂, NH₃, I-, Br-, Cl-, RCOO-, SCN-, ROH, RSH, NCO-, CN-, NO₃-, ClO₄-, H₂O.

18. Verfahren nach Anspruch 16 bis 17, **dadurch gekennzeichnet, dass** die Zusammensetzung, die das oder die in einem der Ansprüche 1 bis 6 definierten Monomere enthält, auf die zuvor befeuchteten Haare aufgebracht wird.

19. Verfahren nach einem der Ansprüche 16 bis 18, **dadurch gekennzeichnet, dass** die Zusammensetzung, die das Monomer enthält, auf Haare aufgebracht wird, die vorab mit Hilfe einer wässrigen Lösung befeuchtet wurden, deren pH-Wert mit einer Base, einer Säure oder einem Säure/Base-Gemisch eingestellt wurde.

20. Verfahren nach Anspruch 16 und 17, **dadurch gekennzeichnet, dass** die Zusammensetzung, die das Monomer enthält, auf Fasern aufgebracht wird, wobei die Fasern mit Hilfe eines von Wasser verschiedenen nucleophilen Stoffes vorimprägniert werden.

21. Verfahren nach einem der Ansprüche 16 bis 20, **dadurch gekennzeichnet, dass** der nucleophile Stoff in reiner Form, als Lösung, in Form einer Emulsion oder eingekapselt verwendet wird.

22. Verfahren nach einem der Ansprüche 16 bis 21, **dadurch gekennzeichnet, dass** nach dem Aufbringen der Zusammensetzung, die das oder die in einem der Ansprüche 1 bis 5 definierten Monomere enthält, ein Spülschritt folgt.
